Europäisches Patentamt

**European Patent Office**

**Office européen des brevets**

(19)

(11) Numéro de publication : **0 049 177**
**B1**

# FASCICULE DE BREVET EUROPÉEN

(12)

(45) Date de publication du fascicule du brevet :
18.01.84

(21) Numéro de dépôt : **81401229.0**

(22) Date de dépôt : **30.07.81**

(51) Int. Cl.³ : **A 61 K   9/70**, A 61 L 15/03

(54) **Pansement comprenant un support textile associé à une composition dermatologique contenant un enzyme et son procédé de préparation.**

(30) Priorité : **19.08.80 FR 8018142**

(43) Date de publication de la demande :
**07.04.82 Bulletin 82/14**

(45) Mention de la délivrance du brevet :
**18.01.84 Bulletin 84/03**

(84) Etats contractants désignés :
**AT BE CH DE FR IT LI**

(56) Documents cités :
**EP-A- 0 013 606**
**FR-M-     6 733**
**CHEMICAL ABSTRACTS, vol. 84, no. 11, 31 mai 1976,**
**page 348, abrégé 15528m Columbus, Ohio, US**
**CHEMICAL ABSTRACTS, vol. 93, no. 22, décembre**
**1980, page 356, abrégé 210109p Columbus, Ohio, US**
**CHEMICAL ABSTRACTS, vol. 94, no. 2, 12 janvier**
**1981, page 254, abrégé 7746x Columbus, Ohio, US**
**Dural, "Dictionnaire de la Chimie et de son applica-**
**tion, 3ème Frd, (1978), p 344**

(73) Titulaire : **LABORATOIRES D'HYGIENE ET DE DIETE-**
**TIQUE L.H.D. Société Anonyme dite:**
**3 rue de Citeaux**
**F-75012 Paris (FR)**

(72) Inventeur : **Guillemet, Alain**
**12 rue Martin de Moinville**
**F-21000 Dijon (FR)**

(74) Mandataire : **Clisci, Serge**
**CABINET BEAU DE LOMENIE 55 rue d'Amsterdam**
**F-75008 Paris (FR)**

# Pansement comprenant un support textile associé à une composition dermatologique contenant un enzyme et son procédé de préparation

La présente invention concerne en tant que produit industriel nouveau un pansement comprenant un support textile associé à une composition dermatologique contenant un enzyme. Elle concerne également son procédé de préparation.

La composition dermatologique comprend de la polyvinyl-pyrrolidone (en abrégé PVP), du glycérol et au moins un enzyme, notamment la catalase.

On sait que l'on a déjà préconisé dans le passé des compositions destinées à être appliquées sur la peau et renfermant, en association avec d'autres substances, de la PVP, du glycérol, et le cas échéant, un principe actif.

On connaît de la demande de brevet FR-A-2 429 251, une composition adhésive utile dans la réalisation de joints à ostomies et comprenant trois ingrédients essentiels : de la glycérine (40 à 70 % en poids), de la polyvinylpyrrolidone (20 à 50 % en poids) et de la carboxyméthylcellulose (5 à 20 % en poids).

On connaît du Brevet Spécial de Médicament FR-A-6 733 M, un matériau folié comprenant notamment une substance filmogène telle que l'alcool polyvinylique (en abrégé PVA), un moyen modifiant les films de PVA tel que la PVP ou le glycérol, et un anesthésique local tel que la lidocaïne.

On connaît aussi de la demande de brevet EP-A-13 606, une matrice de diffusion comprenant une substance filmogène telle que le PVA, de la PVP et du glycérol.

On connaît enfin du résumé des Chemical Abstracts 94, 7746x et du résumé Derwent 55912 C/32 [publié dans Central Patents Index, Country Alerting Bulletin, section B (Farmdoc), Derwent Publications Ltd, Londres (1980)], qui se réfèrent tous deux à la demande de brevet JP-A-80 83710, un matériau spongieux contenant un enzyme, devant adhésif en contact avec l'eau et les liquides corporels, constitué essentiellement d'un polymère hydrosoluble ou formant un gel avec l'eau (en particulier la PVP) en association avec un plastifiant (en particulier le glycérol) et un tensioactif, et étant préparé par lyophilisation.

Selon l'invention, on préconise une nouvelle solution technique pour obtenir à partir de PVP, de glycérol et d'un enzyme une composition dermatologique non adhésive, remplacer les tulles gras dans le traitement des plaies et des dermatoses, et, résoudre le problème du délitage retardé dans le temps d'un enzyme. Cette solution technique qui fait appel à un support textile et une composition dermatologique de PVP, glycérol et enzyme, ledit support et ladite composition étant associés par enduction puis séchage sous courant d'air, est différente de celles de l'art antérieur. Cette nouvelle solution se distingue des solutions connues par le fait que la composition dermatologique préconisée n'est pas adhésive ; elle se distingue en outre, notamment des solutions décrites dans FR-A-6 733 M et EP-A-13 606, par le fait que dans ladite composition dermatologique la PVP remplit une fonction différente de celle qui consistait à modifier le PVA.

Le pansement selon l'invention qui comprend un support textile tissé ou non tissé et une composition dermatologique renfermant de la PVP, du glycérol et un enzyme et qui est utile notamment dans le traitement des plaies et des dermatoses, est caractérisé en ce que le support textile est associé à une composition dermatologique non adhésive comprenant une polyvinyl-pyrrolidone ayant un poids moléculaire compris entre 10 000 et 400 000, du glycérol et un enzyme choisi parmi l'ensemble constitué par la catalase, l'urokinase, la superoxyde-dismutase, l'amylase, les protéases et leurs mélanges, l'association du support textile avec la composition dermatologique étant obtenue par enduction par voie humide puis séchage sous courant d'air, le rapport polyvinylpyrrolidone-glycérol dans ladite composition dermatologique étant compris entre (3 : 1) et (5 : 1) en poids sec.

Avec la PVP qui est un polymère hydrosoluble et le glycérol qui est un plastifiant également hydrosoluble, on obtient une base dermatologique qui n'est pas grasse, qui est non adhésive, et qui est utile en association avec le support textile pour le traitement des plaies et des dermatoses, en vue de remplacer les tulles gras (en raison de ses propriétés protectrice, adsorbante des sérosités et non adhérente), et susceptible d'être anti-inflammatoire quand elle est perméable et non anti-inflammatoire dans le cas contraire. Selon l'invention les enzymes conservent leurs activités (ce qui est particulièrement intéressant pour les produits enzymatiques, tels que la catalase, qui voient leur activité diminuer au contact de l'air) et sont libérés au contact de la peau par la solubilisation de la PVP et du plastifiant par les liquides corporels.

Les PVP qui conviennent ont un poids moléculaire compris entre 10 000 et 400 000. Le moyen préféré est la PVP ayant un poids moléculaire de l'ordre de 40 000.

Dans la composition dermatologique le rapport pondéral PVP-glycérol est selon l'invention compris entre (3 : 1) et (5 : 1) et, de préférence compris entre (3,5 : 1) et (4 : 1).

L'enzyme préféré pour isoler et cicatriser les plaies est la catalase. Le cas échéant, la composition dermatologique peut également renfermer un antiseptique, tel que l'hexamidine ou le diiséthionate d'hexamidine.

La méthode de préparation de la composition dermatologique consiste à mélanger la PVP, le glycérol et l'enzyme par voie humide et à évaporer l'eau.

Plus précisément, on préconise de préparer un premier mélange comprenant la substance enzymatique et une partie de la PVP, d'ajouter audit mélange ainsi obtenu le reste de la PVP et le glycérol.

Le meilleur mode de mise en œuvre (spécialement préconisé quand l'enzyme est un principe actif fragile, tel que la catalase) du procédé de préparation du pansement selon l'invention consiste :

1) à mélanger par voie humide l'enzyme et une partie de la PVP,

2) à éliminer l'eau pour obtenir une poudre,

3) à mélanger la poudre ainsi obtenue avec le reste de la PVP et le glycérol, dans de l'eau, sous agitation, pour obtenir une composition homogène,

4) à associer la composition résultante au support textile par enduction, puis

5) à éliminer l'eau par séchage sous courant d'air pour obtenir un produit sec.

L'élimination de l'eau est réalisée par lyophilisation ou évaporation à la pression atmosphérique à 40-90 °C au stade 2) et par évaporation à 40-90 °C au stade 5). De préférence, la température d'évaporation est de 80 °C.

Le pansement selon l'invention pourra être conditionné de façon à être soit occlusif, soit non occlusif.

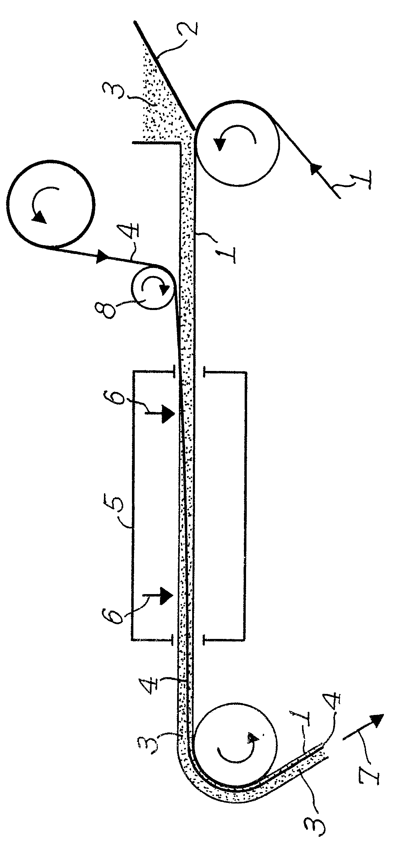

On a représenté schématiquement dans la figure 1 le mécanisme de préparation d'un support textile enduit d'une composition dermatologique selon l'invention. Selon ce mécanisme, on dépose sur un support antiadhérent 1 (de préférence un papier siliconé pour avoir un support temporaire), au moyen d'un dispositif d'enduction 2 (notamment du type lame ou racle), une composition aqueuse comprenant le mélange 3 constitué par la PVP, le glycérol et l'enzyme ; on amène un textile 4 (tissé ou, de préférence, non tissé), au moyen d'un cylindre 8 réglable en hauteur, au contact de la masse 3 déposée sur le support 1, puis on évapore l'eau au moyen d'un four 5 muni d'arrivées d'air 6. A la sortie du four, le produit, qui va être recueilli en 7, est tel que la masse 3 sèche est située de part et d'autre du support 4 (au cours du séchage, ledit support s'est enfoncé dans ladite masse). On obtient ainsi un dépôt de 20 à 250 g/m² de composition dermatologique.

D'autres avantages et caractéristiques de l'invention seront mieux compris à la lecture qui va suivre d'exemples de mise en œuvre nullement limitatifs.

Exemple 1

Pansement à base de catalase.

1) On prépare par voie humide un mélange PVP-catalase (3 : 1) en poids dans lequel la PVP a un poids moléculaire d'environ 40 000.

2) On lyophilise le mélange ainsi obtenu.

3) 40 parties en poids de la poudre lyophilisée ainsi obtenue sont mélangées à 40 parties en poids de PVP de poids moléculaire de l'ordre de 40 000 et à 20 parties en poids de glycérol ; on agite le mélange résultant avec de l'eau (100 parties en poids).

4) Un support textile (non tissé) est enduit avec le mélange comprenant 100 parties en poids d'eau, 40 parties en poids du mélange pondéral PVP-catalase (3 : 1), 40 parties en poids de PVP et 20 parties en poids de glycérol (c'est-à-dire un mélange renfermant au total 100 parties en poids d'eau, 70 parties en poids de PVP, 10 parties en poids de catalase et 20 parties en poids de glycérol), selon le mécanisme schématisé par la figure 1.

5) On passe alors dans un four tunnel à 80 °C pour évaporer l'eau sous courant d'air. Par enduction, la quantité de composition en matière sèche est comprise entre 20 et 250 g/m² et avantageusement de 100 g/m² (soit 10 g/m² d'enzyme).

Le pansement obtenu selon cette technique est particulièrement indiqué en tant que produit cicatrisant.

Exemple 2

Pansement à base de catalase.

On procède comme indiqué à l'exemple 1, en remplaçant la lyophilisation du stade 2) par un séchage sous pression atmosphérique. On obtient un produit analogue à celui de l'exemple 1.

Exemple 3

Pansement à base de catalase.

1) On prépare par voie humide un mélange de 35 parties en poids de PVP ayant un poids moléculaire d'environ 40 000 et de 5 parties en poids de catalase.

2) On évapore l'eau sous pression atmosphérique.

3) Le mélange résultant est mélangé à 40 parties en poids de PVP ayant un poids moléculaire de l'ordre de 40 000 et 20 parties en poids de glycérol, et on agite avec 100 parties en poids d'eau.

On procède ensuite à une enduction puis à un séchage comme indiqué à l'exemple 1.

Exemple 4

Pansement à base de catalase et d'hexamidine.

On procède comme indiqué à l'exemple 3 en remplaçant au stade 1) les 35 parties en poids de PVP et les 5 parties en poids de catalase par 34,5 parties en poids de PVP (ayant un poids moléculaire de l'ordre de 40 000) 4,5 parties en poids de catalase et 1 partie en poids d'hexamidine.

## Exemple 5

Pansement à base de catalase.

On procède comme indiqué à l'exemple 3, en utilisant au stade 1) 36 parties en poids de PVP (ayant un poids moléculaire de l'ordre de 40 000) et 10 parties en poids de catalase, et au stade 3) 36 parties en poids de PVP (ayant un poids moléculaire de l'ordre de 40 000) et 18 parties en poids de glycérol.

## Exemple 6

Pansement à base de catalase et d'un moyen antiseptique.

1) On prépare par voie humide un mélange à partir de 34,5 parties en poids de PVP (ayant un poids moléculaire de environ 40 000), de 4,5 parties en poids de catalase ayant un titre de $4,5 \times 10^6$ UI/g et de 1 partie en poids d'un principe actif (diiséthionate d'hexamidine).

2) On évapore l'eau sous pression atmosphérique.

3) Le produit résultant de l'évaporation de l'eau est mélangé sous agitation avec 40 parties en poids de PVP (ayant un poids moléculaire de environ 40 000) et 20 parties en poids de glycérol, et 100 parties en poids d'eau.

4) Le mélange résultant est déposé par enduction sur un support (tissé ou non tissé) de façon que après séchage dans un four tunnel à 80 °C sous courant d'air on obtienne un dépôt d'enduction de 100 g/m² comprenant 74,5 g/m² de PVP, 4,5 g/m² de catalase, 1 g/m² de diiséthionate d'hexamidine et 20 g/m² de glycérol. Le pansement ainsi obtenu comprend donc 2 025 UI/cm² de catalase.

## Revendications

1. Pansement comprenant un support textile tissé ou non tissé et une composition dermatologique renfermant de la polyvinylpyrrolidone, du glycérol et un enzyme, utile notamment pour le traitement des plaies et des dermatoses, caractérisé en ce que le support textile est associé à une composition dermatologique non adhésive comprenant une polyvinylpyrrolidone ayant un poids moléculaire compris entre 10 000 et 400 000, du glycérol et un enzyme choisi parmi l'ensemble constitué par la catalase, l'urokinase, la superoxyde-dismutase, l'amylase, les protéases et leurs mélanges, l'association du support textile avec la composition dermatologique étant obtenue par enduction par voie humide puis séchage sous courant d'air, le rapport polyvinyl-pyrrolidone-glycérol dans ladite composition dermatologique étant compris entre (3 : 1) et (5 : 1) en poids sec.

2. Pansement selon la revendication 1, caractérisé en ce que la polyvinylpyrrolidone de la composition dermatologique a un poids moléculaire de 40 000 environ.

3. Pansement selon la revendication 1, caractérisé en ce que dans la composition dermatologique le rapport pondéral polyvinylpyrrolidone-glycérol est compris entre (3,5 : 1) et (4 : 1).

4. Pansement selon la revendication 1, caractérisé en ce que l'enzyme contenu dans la composition dermatologique est la catalase.

5. Pansement selon la revendication 1, caractérisé en ce que le support textile est logé dans l'épaisseur de la composition dermatologique.

6. Pansement selon la revendication 1, caractérisé en ce que la composition dermatologique comprend, outre la PVP, le glycérol et l'enzyme, de l'hexamidine en tant qu'agent antiseptique.

7. Procédé de préparation d'un pansement comprenant un support textile associé par enduction à une composition dermatologique selon la revendication 1, caractérisé en ce que :

1) on mélange par voie humide l'enzyme et une partie de la polyvinylpyrrolidone,

2) on élimine l'eau pour obtenir une poudre,

3) on mélange la poudre ainsi obtenue avec le reste de la polyvinylpyrrolidone et le glycérol dans de l'eau, sous agitation, pour obtenir une composition homogène,

4) on associe la composition résultante au support textile par enduction, puis

5) on élimine l'eau par séchage sous courant d'air pour obtenir un produit sec.

8. Procédé selon la revendication 7, caractérisé en ce que l'enzyme est la catalase, et en ce que l'élimination de l'eau est réalisée au stade 2) par lyophilisation et au stade 5) par évaporation à 40-90 °C sous courant d'air.

9. Procédé selon la revendication 7, caractérisé en ce que l'enzyme est la catalase, et en ce que l'élimination de l'eau est réalisée au stade 2) et au stade 5) par évaporation à 40-90 °C sous courant d'air.

10. Procédé selon l'une quelconque des revendications 8 et 9, caractérisé en ce que l'évaporation de l'eau sous courant d'air est effectuée à 80 °C sous pression atmosphérique.

## Claims

1. A dressing comprising a woven or non-woven fabric support and a dermatological composition containing polyvinylpyrrolidone, glycerol and an enzyme, useful particularly in the treatment of wounds and dermatoses, characterised in that the fabric support is associated with a non-adhesive dermatological composition comprising a polyvinylpyrrolidone having a molecular weight comprised between 10,000 and 400,000, glycerol and an enzyme selected from the group consisting of catalase, urokinase, superoxide dismutase, amylase, proteases and mixtures thereof, the association of the fabric support with the dermatological composition being obtained by wet coating then draught drying, and the polyvinyl-pyrrolidone-glycerol ratio in said dermatological composition being comprised between (3 : 1) and (5 : 1) by dry weight.

2. A dressing according to claim 1, characterised in that the polyvinylpyrrolidone of said dermatological composition has a molecular weight of about 40,000.

3. A dressing according to claim 1, characterised in that in said dermatological composition, the polyvinylpyrrolidone-glycerol ratio is comprised between (3.5 : 1) and (4 : 1) by dry weight.

4. A dressing according to claim 1, characterised in that the enzyme contained in said dermatological composition is catalase.

5. A dressing according to claim 1, characterised in that the fabric support is housed in the width of said dermatological composition.

6. A dressing according to claim 1, characterised in that said dermatological composition, besides said polyvinylpyrrolidone, glycerol and enzyme, also contains hexamidine as an antiseptic means.

7. A method for preparing a dressing comprising a fabric support and a dermatological composition associated with said fabric support by coating according to claim 1, characterised in that it comprises the following steps :

1) mixing in water the enzyme and a portion of the polyvinylpyrrolidone,

2) eliminating water in order to obtain a powder,

3) mixing in water the powder thus obtained, the glycerol and the remaining portion of the polyvinylpyrrolidone,

4) associating the resulting composition to the fabric support by coating, and

5) eliminating water by draught drying in order to obtain a dry product.

8. A method according to claim 7, characterised in that the enzyme is catalase, and in that water elimination is carried out by lyophilisation in step 2) and by draught drying at 40-90 °C in step 5).

9. A method according to claim 7, characterised in that the enzyme is catalase, and in that water elimination is carried out by draught drying at 40-90 °C in steps 2) and 5).

10. A method according to claim 8 or 9, characterised in that water elimination is carried out by draught drying at 80 °C under atmospheric pressure.

## Ansprüche

1. Verband mit einem gewebten oder nichtgewebten textilen Trägermaterial und einer Polyvinylpyrrolidon, Glycerin und ein Enzym enthaltenden dermatologischen Zusammensetzung, welcher insbesondere zur Behandlung von Wunden und Dermatosen geeignet ist, dadurch gekennzeichnet, daß das textile Trägermaterial mit einer nichthaftenden dermatologischen Zusammensetzung, enthaltend Polyvinylpyrrolidon mit einem Molekulargewicht zwischen 10 000 und 400 000, Glycerin und ein Enzym, ausgewählt aus der Gruppe umfassend Katalase, Urokinase, Superoxid-Dismutase, Amylase, Proteasen und Mischungen davon, verbunden ist, wobei die Verbindung des textilen Trägermaterials mit der dermatologischen Zusammensetzung durch Beschichtung auf feuchtem Wege und anschließende Trocknung unter einem Luftstrom erzielt wird und das Verhältnis Polyvinylpyrrolidon-Glycerin in der dermatologischen Zusammensetzung zwischen (3 : 1) und (5 : 1) Trockengewicht beträgt.

2. Verband nach Anspruch 1, dadurch gekennzeichnet, daß das Molekulargewicht des Polyvi-nylpyrrolidons der dermatologischen Zusammen-setzung etwa 40 000 beträgt.

3. Verband nach Anspruch 1, dadurch gekenn-zeichnet, daß das Gewichtsverhältnis Polyvi-nylpyrrolidon-Glycerin in der dermatologischen Zusammensetzung zwischen (3,5 : 1) und (4 : 1) beträgt.

4. Verband nach Anspruch 1, dadurch gekennzeichnet, daß das in der dermatologischen Zusammensetzung enthaltene Enzym Katalase ist.

5. Verband nach Anspruch 1, dadurch gekennzeichnet, daß das textile Trägermaterial in der Schichtstärke der dermatologischen Zusammensetzung eingebettet ist.

6. Verband nach Anspruch 1, dadurch gekenn-zeichnet, daß die dermatologische Zusammen-setzung neben PVP, Glycerin und dem Enzym Hexamidin als antiseptisches Mittel enthält.

7. Verfahren zur Herstellung eines Verbandes nach Anspruch 1, umfassend ein textiles Trägermaterial, das durch Beschichtung mit einer dermatologischen Zusammensetzung verbunden ist, dadurch gekennzeichnet, daß :

1) das Enzym und ein Teil des Polyvinylpyrrolidons auf feuchtem Wege vermischt werden,

2) das Wasser zur Gewinnung eines Pulvers entfernt wird,

3) das so erhaltene Pulver mit dem restlichen Polyvinylpyrrolidon und dem Glycerin in Wasser zur Gewinnung einer homogenen Zusammensetzung unter Rühren vermischt wird,

4) die resultierende Zusammensetzung durch Beschichtung mit dem textilen Trägermaterial verbunden wird, und

5) das Wasser durch Trocknung unter einem Luftstrom zur Gewinnung eines trockenen Produktes entfernt wird.

8. Verfahren nach Anspruch 7, dadurch gekennzeichnet, daß das Enzym Katalase ist und die Entfernung des Wassers in der Stufe 2) durch Lyophilisieren und in der Stufe 5) durch Verdampfen bei 40 bis 90 °C unter einem Luftstrom erfolgt.

9. Verfahren nach Anspruch 7, dadurch gekennzeichnet, daß das Enzym Katalase ist und die Entfernung des Wassers in der Stufe 2) und in der Stufe 5) durch Verdampfen bei 40 bis 90 °C unter einem Luftstrom erfolgt.

10. Verfahren nach einem der Ansprüche 8 und 9, dadurch gekennzeichnet, daß die Verdampfung des Wassers unter einem Luftstrom bei 80 °C unter Atmosphärendruck erfolgt.

6  5  6

3  4  8  4  3

2

1

1

3  1

4

7